Europäisches Patentamt

European Patent Office

Office européen des brevets

(11) Veröffentlichungsnummer: **0 122 367**
A2

## (12) EUROPÄISCHE PATENTANMELDUNG

(21) Anmeldenummer: **84100485.6**

(22) Anmeldetag: **18.01.84**

(51) Int. Cl.³: **B 01 D 3/16**

(30) Priorität: **26.01.83 DE 3302525**

(43) Veröffentlichungstag der Anmeldung:
**24.10.84 Patentblatt 84/43**

(84) Benannte Vertragsstaaten:
**BE CH DE FR GB IT LI NL**

(71) Anmelder: **BASF Aktiengesellschaft
Carl-Bosch-Strasse 38
D-6700 Ludwigshafen(DE)**

(72) Erfinder: **Kaibel, Gerd
Robert-Bosch-Strasse 4
D-6840 Lampertheim 1(DE)**

(54) Destillationskolonne.

(57) Destillationskolonne zur destillativen Zerlegung eines an einer Zulaufstelle in die Destillationskolonne eintretenden Zulaufproduktes, bestehend aus mehreren Fraktionen, in eine reine Kopf- und eine reine Sumpffraktion und mehrere, vorzugsweise eine oder zwei, im Siedebereich zwischen Kopf- und Sumpffraktion liegende und von Verunreinigungen durch Kopf- und Sumpffraktionen freie oder weitgehend freie Mittelsiederfraktionen, wobei in einem Teilbereich der Destillationskolonne unterhalb und/oder oberhalb der Zulaufstelle in Längsrichtung wirksame Trenneinrichtungen sur Verhinderung einer Quervermischung von Flüssigkeits- und/oder Brüdenströmen angeordnet sind, die die Destillationskolonne in einen Zulaufteil, in den das Zulaufprodukt eintritt, und einen Entnahmeteil, aus dem die Mittelsiederfraktionen austreten, unterteilen, und daß die in Längsrichtung wirksamen Trenneinrichtungen über so viele Trennstufen ausgeführt sind, daß im Entnahmeteil die von Verunreinigungen durch Kopfund Sumpffraktionen freien oder weitgehend freien Mittelsiederfraktionen abgezogen werden können.

FIG.1

FIG.2

BEZEICHNUNG GEÄNDERT
siehe Titelseite

Destillationskolonne zur destillativen Zerlegung eines aus mehreren
Fraktionen bestehenden Zulaufproduktes

Die Erfindung betrifft eine Destillationskolonne zur destillativen Zerlegung eines an einer Zulaufstelle in die Destillationskolonne eintretenden Zulaufproduktes, bestehend aus mehreren Fraktionen, in eine reine
Kopf- und eine reine Sumpffraktion und mehrere, vorzugsweise eine oder
zwei, im Siedebereich zwischen Kopf- und Sumpffraktion liegende und von
Verunreinigungen durch Kopf- und Sumpffraktionen freie oder weitgehend
freie Mittelsiederfraktionen.

Bei der kontinuierlichen Trennung eines aus n Fraktionen bestehenden
Stoffgemisches sind zur Zerlegung in die reinen Fraktionen n-1 Destillationsschritte erforderlich. Dies erfordert bei Stoffgemischen, die sich
aus zahlreichen Fraktionen zusammensetzen, einen hohen technischen Aufwand.

Das in der Industrie, beispielsweise in Raffinerien, häufig angewandte
Verfahren zur kontinuierlichen destillativen Trennung eines Stoffgemisches in seine Fraktionen ist das Verfahren der fraktionierten Destillation mittels einer Destillationskolonne (im folgenden Kolonne genannt),
bei dem ein Stoffgemisch aus n Fraktionen in eine Kopf- und eine Sumpffraktion und n-2 Seitenfraktionen zerlegt wird. Mittels dieses Verfahrens
ist es zwar möglich, reine Kopf- und reine Sumpffraktionen zu gewinnen,
die Seitenfraktionen sind jedoch stets verunreinigt. Im Verstärkungsteil
der Kolonne sind die Seitenfraktionen mit der leichtest siedenden Komponente verunreinigt, da diese an der Seitenabzugsstelle vorbeitransportiert werden muß. Man kann die Menge der Verunreinigungen durch die
leichtest siedende Komponente zwar dadurch gering halten, daß man Seitenabzüge im Verstärkunsteil in flüssiger Form vornimmt. Entsprechend dem
Dampf-/Flüssigkeitsgleichgewicht enthält die flüssige Phase weniger der
leichtest siedenden Komponente als die Dampfphase, jedoch kann eine
völlige Reinheit der Seitenfraktion nie erreicht werden. Analog gilt dies
für den Abtriebsteil der Kolonne, wo man aus den vorgenannten Gründen die
Seitenfraktionen dampfförmig abzieht, jedoch - abgesehen von nicht destillierbaren Stoffen, wie beispielsweise Salzen - ebenfalls keine völlige
Reinheit der Seitenfraktionen erzielen kann.

Eine andere Möglichkeit zur Verringerung des Trennaufwandes bietet der
Einsatz einer Hauptkolonne mit angeschlossenen Seitenkolonnen. Hierbei
benötigt jede Seitenentnahme eine eigene Seitenkolonne. Im Vergleich zur

Go/P

Ausführung mit völlig getrennten Kolonnen wird jedoch für jede Seitenentnahme ein Kondensator bzw. ein Verdampfer eingespart.

Eine weitere Möglichkeit zur Erhöhung der Reinheit der Seitenfraktionen bietet eine Vergrößerung der Heizleistung, da dann infolge des höheren Rücklaufverhältnisses eine stärkere Verdünnung des Leichtersieders an den Seitenabzugsstellen im Verstärkungsteil und des Schwerersieders an den Seitenabzugsstellen im Abtriebsteil der Kolonne eintritt. Diese bessere Produktreinheit muß jedoch durch höhere Energiekosten erkauft werden und scheidet meist aus wirtschaftlichen Gründen aus. Zudem kann durch diese Maßnahme auch bei Aufwendung größter Energiemengen nie eine völlige Reinheit der Seitenfraktionen erreicht werden.

Der Erfindung liegt die Aufgabe zugrunde, die vorgenannten Nachteile zu vermeiden.

Diese Aufgabe wird erfindungsgemäß dadurch gelöst, daß in einem Teilbereich der Destillationskolonne unterhalb und/oder oberhalb der Zulaufstelle in Längsrichtung wirksame Trenneinrichtungen zur Verhinderung einer Quervermischung von Flüssigkeits- und/oder Brüdenströmen angeordnet sind, die die Destillationskolonne in einen Zulaufteil, in den das Zulaufprodukt eintritt, und einen Entnahmeteil, aus dem die Mittelsiederfraktionen austreten, unterteilen, und daß die in Längsrichtung wirksamen Trenneinrichtungen über so viele Trennstufen ausgeführt sind, daß im Entnahmeteil die von Verunreinigungen durch Kopf- und Sumpffraktionen freien oder weitgehend freien Mittelsiederfraktionen abgezogen werden können.

Ein Ausführungsbeispiel der Erfindung ist in der Zeichnung dargestellt und wird im folgenden näher beschrieben.

Es zeigen

Figur 1    ein schematisches Verfahrensfließbild einer Kolonne zur destillativen Zerlegung eines Stoffgemisches aus 3 Fraktionen in reine Fraktionen mittels der erfindungsgemäßen Trenneinrichtung,

Figur 2    ein schematisches Verfahrensfließbild einer Kolonne zur destillativen Zerlegung eines Stoffgemisches aus 4 Fraktionen in reine Fraktionen mittels der erfindungsgemäßen Trenneinrichtung,

Figuren 3
und 4      jeweils ein schematisches Verfahrensfließbild einer Kolonne zur destillativen Zerlegung eines Stoffgemisches aus mehreren Fraktionen in reine Fraktionen mittels der erfindungsgemäßen Trenneinrichtung.

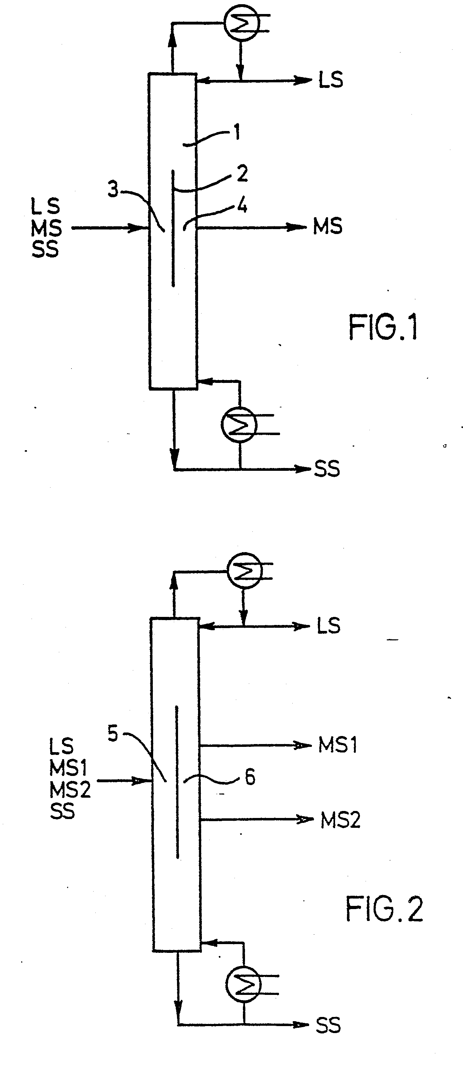

Gemäß Figur 1 wird eine Kolonne 1 durch eine in Längsrichtung wirksame Trenneinrichtung 2 unterhalb und oberhalb der Zulaufstelle in einen Zulaufteil 3 und einen Entnahmeteil 4 unterteilt. Der Mittelsieder wird aus dem Zulaufteil über dessen Verstärkungsteil und/oder dessen Abtriebsteil herausdestilliert. Es ist dabei beliebig, über welchen Teil der Zwischensieder aus dem Zulaufteil herausdestilliert wird. Entscheidend ist nur, daß aus dem Zulaufteil kein Schwerersieder nach oben und kein Leichtersieder nach unten herausdestilliert wird, was bei entsprechender Dimensionierung dieses Kolonnenteils möglich ist. Durch diese Maßnahmen wird erreicht, daß im Verstärkungsteil des Entnahmeteils kein Schwerersieder und im Abtriebsteil des Entnahmeteils kein Leichtersieder vorhanden ist. Hierdurch kann man an der Entnahmestelle, die in Höhe der Zulaufstelle aber auch höher oder tiefer liegen kann, eine reine Fraktion des Mittelsieders entnehmen. Im Gegensatz zu einer konventionellen Seitenabzugskolonne ist es hierbei unerheblich, ob der Seitenabzug in flüssiger oder dampfförmiger Form entnommen wird.

Wie Figur 2 zeigt, ist es bei der gleichen Anordnung auch möglich, ein Stoffgemisch aus 4 Fraktionen in 4 reine Fraktionen zu zerlegen. In diesem Fall wird der Verstärkungsteil des Zulaufteiles 5 so dimensioniert, daß der Mittelsieder 2 und der Schwerersieder nicht nach oben wegdestillieren können. Der Abtriebsteil des Zulaufteils verhindert, daß der Leichtersieder sowie der Mittelsieder 1 nach unten abgetrennt werden. Im Entnahmeteil 6 kann daher die Trennung der Mittelsieder in zwei reine Fraktionen erfolgen.

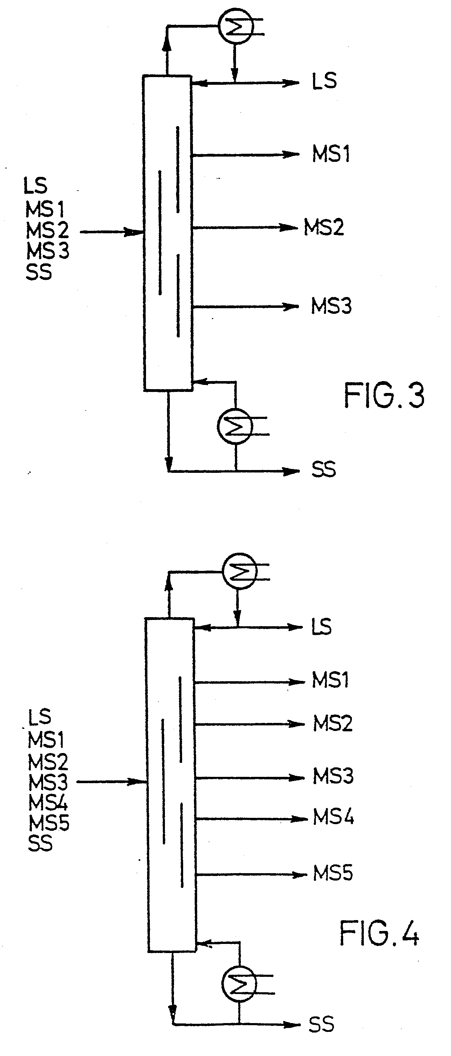

Die beiden in Figur 1 und Figur 2 dargestellten Beispiele zeigen das Grundprinzip bei der Verwendung von in Längsrichtung wirksamen Trenneinrichtungen und der dadurch erreichten Unterteilung der Kolonne in einen Zulauf- und einen Entnahmeteil. Dieses Grundprinzip läßt sich durch Zufügen weiterer Trenneinrichtungen ausdehnen und ermöglicht somit auch prinzipiell die Auftrennung von Gemischen in beliebig viele reine Fraktionen. Dabei entspricht das obere und/oder das untere Ende der Trenneinrichtung jeweils einer neuen Zulaufstelle. Mögliche Beispiele sind in den Figuren 3 und 4 dargestellt. In der technischen Anwendung ist die Zahl der Fraktionen dadurch praktisch begrenzt, daß mit zunehmender Zahl der Fraktionen auch die Zahl der benötigten Trennstufen ansteigt, was wegen der Kolonnenbauhöhe, des Druckverlustes längs der Kolonne und der damit oft zu hohen Sumpftemperaturen Grenzen setzt.

Die Trennstufenzahl einer solchen Kolonne entspricht etwa der einer konventionellen Seitenabzugskolonne, ist jedoch niedriger als bei einer Hauptkolonne mit Seitenkolonnen, wenn man die Gesamttrennstufenzahl ver-

0122367

gleicht. Auch hinsichtlich des Energiebedarfes liegt die erfindungsgemäße Kolonne mit Zulauf- und Entnahmeteil günstiger als eine Kolonne mit Seitenkolonnen. Zudem ist als Vorteil zu werten, daß nur jeweils 1 Verdampfer sowie 1 Kondensator benötigt werden, während Seitenkolonnen eigene Verdampfer bzw. Kondensatoren erfordern.

Die Längsunterteilung im Bereich der Zulaufstelle verschiedener Kolonnen wird im nachfolgenden näher beschrieben; beispielsweise ist die Längsunterteilung bei einer Füllkörperkolonne oder einer Kolonne mit Packungen besonders einfach. Hier genügt ein durchgehendes Trennblech zwischen den Kolonnenwänden den Erfordernissen. Bei einer Kolonne mit Regensiebböden kann die Unterteilung zwischen den einzelnen Regensiebböden beliebig angebracht werden. Voraussetzung ist, daß der Zwischenraum zwischen zwei Regensiebböden in zwei völlig unabhängige Segmente unterteilt wird. Bei einer Kolonne mit Glockenböden, Ventilböden oder Siebböden jeweils mit einem Ablaufschacht ist es erforderlich, die Trennwand durch den gesamten Ablaufschacht zu legen. Bei den oben erwähnten Böden, die infolge großer Abmessungen mehrflutig, d.h. also jeder Boden mit zwei oder mehreren Ablaufschächten ausgerüstet ist, kann die Längsunterteilung beliebig angeordnet werden.

Die Auslegung einer solchen Kolonne bezüglich Heizleistung, Trennstufenzahl, Anordnung von Zulauf- und Seitenentnahmestellen sowie Länge der in Längsrichtung wirksamen Trennvorrichtung (Unterteilung) kann wie bei einer Kolonne ohne Längsunterteilung rechnerisch oder experimentell erfolgen.

Bei Kolonnen mit nur einer Seitenentnahme und etwa gleich großen Siedepunktsdifferenzen der Seitenabzugsfraktion zur Kopf- und Sumpffraktion kann man die Zulauf- und die Entnahmestelle auf gleicher Höhe anordnen. Wenn diese Siedepunktsdifferenzen jedoch sehr ungleich sind, ist es günstiger die Zulauf- und die Entnahmestelle auf verschiedener Höhe anzubringen.

Geringe Siedepunktsunterschiede der zu trennenden Fraktionen und hohe Reinheitsanforderungen an die Seitenfraktion erfordern lange Unterteilungen. Bei geringeren Anforderungen an die Trennschärfe genügen kürzere Trennvorrichtungen. Gegebenenfalls kann die Zulauf- oder Entnahmestelle auch am Ende der Trennvorrichtung angeordnet werden. Es ist bei verringerten Reinheitsansprüchen auch möglich, auf eine völlige Verhinderung der Quervermischung zu verzichten und einfachere Trennvorrichtungen vorzusehen, die beispielsweise infolge unvollständiger Abdichtung die Quervermischung von Flüssigkeit und/oder Brüden nur teilweise unterdrücken.

Insbesondere bei Füllkörperkolonnen und Kolonnen mit Packungen kann es bei großen Siedepunktsdifferenzen der zu trennenden Fraktionen zweckmäßig sein, die Trennvorrichtung konstruktiv so auszubilden, daß sich eine Wärmedämmung ergibt, um eine die Trennwirksamkeit beeinträchtigende Ungleichverteilung der Flüssigkeit durch eine unkontrollierte Verdampfung bzw. Kondensation zu vermeiden. Im Falle von Trennblechen können diese z.B. doppelwandig mit einer innenliegenden wärmeisolierenden Schicht ausgeführt werden. Gegebenenfalls können die Trenneinbauten auch beheizt und/oder gekühlt werden.

Die Geometrie der Trenneinrichtung kann verschieden sein. So kann der Zulauf- oder Entnahmeteil z.B. als konzentrisches Innenrohr ausgebildet sein. Im allgemeinen sind jedoch als einfachste Ausführungsform ebene Trennbleche zu bevorzugen. Bei Bodenkolonnen mit großen Durchmessern bezieht man die Trennvorrichtung zweckmäßigerweise so in die Konstruktion ein, daß die Trennwände zur Festigkeit der Böden beitragen und eine leichtere Bauweise ermöglichen.

Der optimierte Betrieb einer Kolonne mit Längsunterteilung verlangt eine für jedes Trennproblem spezifische Aufteilung des Brüden- und des Flüssigkeitsstroms auf den Zulauf- und den Entnahmeteil. Dabei bietet die gezielte Aufteilung der Flüssigkeit am oberen Ende der Längsunterteilung keine besondere technische Schwierigkeiten, da sie analog zu der Flüssigkeitsaufteilung in Rücklauf und Ablauf am Kolonnenkopf vorgenommen werden kann.

Die Aufteilung des Brüdenstroms am unteren Ende der Trennvorrichtung wird durch das Flächenverhältnis von Zulauf- und Entnahmeteil beeinflußt. Wie Destillationsrechnungen zeigen, kann die Aufteilung des Brüdenstroms in den meisten Fällen in einem weiten Bereich von etwa 3 : 1 bis 1 : 3 variiert werden, ohne daß nennenswerte Nachteile hinsichtlich Energiebedarf oder Reinheit der Fraktionen auftreten, da Abweichungen in der Brüdenaufteilung durch entsprechende Änderungen der Flüssigkeitsaufteilung am oberen Ende der Längsunterteilung leicht kompensiert werden können. Daher kann man die Längsunterteilung in den meisten Fällen so anordnen, daß die Querschnittsfläche der Kolonne und damit annähernd auch der Brüdenstrom halbiert werden.

Eine Beeinflussung der Brüdenaufteilung durch besondere Maßnahmen ist nur in Sonderfällen angezeigt, wenn z.B. die Strömungswiderstände im Zulauf- und Entnahmeteil stark voneinander abweichen. Dies ist beispielsweise dann der Fall, wenn verschiedene Trennstufenzahlen oder verschiedene Einbauten mit unterschiedlichem Druckverlust (Gewebepackungen, Füllkörper, Böden) vorhanden sind. Hier ist es zweckmäßig im Zulauf- und/oder Entnahmeteil zusätzliche Maßnahmen zur Beeinflussung der Strömungswiderstände vorzusehen, wie Blenden, Drosselklappen oder Destillationsböden mit hohem veränderbarem Flüssigkeitsstand.

An das Betreiben einer Kolonne mit Längsunterteilung müssen keine besonderen Anforderungen gestellt werden. Die Produktreinheiten werden wie bei konventionellen Seitenabzugskolonnen über Temperaturen in der Kolonne geregelt, wobei bei längsunterteilten Kolonnen die Seitenabzugsmenge bevorzugt über Temperaturen im Entnahmeteil gesteuert wird. Im allgemeinen genügt es, die Brüdenaufteilung grob einzustellen z.B. auf den Wert 1 : 1. Auch die Flüssigkeitsaufteilung muß nicht genau gesteuert bzw. geregelt werden. Es genügt, sie auf einen festen Wert einzustellen, wobei es sich in der Regel empfiehlt, mehr Flüssigkeit in den Entnahme- als in den Zuaufteil zu geben. Wie Versuche in einer Kolonne im Labormaßstab zeigten, bringt eine Kolonne mit Längsunterteilung auch bei Brüden- und Flüssigkeitsaufteilungen, die beide willkürlich auf den (nicht optimalen) Wert 1 : 1 festgelegt wurden, Produktreinheiten, die über die mit konventionellen Seitenabzugskolonnen ohne Längsunterteilung erreichbaren weit hinausgehen.

Beispiel
Trennung eines n-Hexan/n-Heptan/n-Octan-Gemisches

Die Trennung erfolgte bei Normaldruck in einer schutzbeheizten Füllkörperkolonne mit einem Innendurchmesser von 50 mm. Als Füllkörper wurden Maschendrahtringe mit 5 mm Durchmesser verwendet. Die Gesamtschütthöhe betrug 2,20 m und ergab eine theoretische Trennstufenzahl von etwa 50 Böden.

Zwischen dem 20. und 40. Boden (von unten gerechnet) befand sich eine senkrecht angeordnete 3 mm dicke ebene Trennwand aus PTFE-Kunststoff, die die Kolonne in zwei Teile mit gleicher Querschnittsfläche unterteilte. In diesem Bereich befanden sich auf Höhe des 32. Bodens sowohl die Zulaufstelle als auch eine Seitenabzugsstelle für Flüssigkeit. Am Kopf der Kolonne sowie oberhalb der Trennwand waren magnetisch betätigte Schwenktrichter zur Aufteilung der Flüssigkeit angebracht. Das Rücklaufverhältnis am Kopf der Kolonne betrug 4 : 1. Oberhalb der Längsunterteilung wurde die Flüssigkeit im Mengenverhältnis 2,5 : 1 auf den Entnahme- und den Zulaufteil aufgeteilt.

Das zu trennende Gemisch bestand aus einer äquimolaren Mischung von n-Hexan, n-Heptan und n-Octan, die in einer Menge von 200 g/h bei einer Temperatur von 70°C flüssig in die Destillationskolonne eingespeist wurde.

Als Kopfprodukt wurden bei 68°C ca. 57 g/h Flüssigkeit entnommen. Die gaschromatographische Analyse ergab einen Gehalt von 99,9 % n-Hexan. Am Kolonnensumpf (126°C) fielen etwa 76 g/h n-Octan in einer Reinheit von über 99,9 % an. Das bei einer Temperatur von ca. 98°C flüssig entnommene Seitenprodukt (etwa 67 g/h) enthielt ca. 99,3 % n-Heptan, 0,3 % n-Hexan und 0,4 % n-Octan.

Die Nachrechnung einer bezüglich Heizleistung und Trennstufenzahl vergleichbaren Seitenabzugskolonne ohne Längsunterteilung ergab, daß bei gleicher Kopf- und Sumpfreinheit für den Seitenabzug eine Reinheit von maximal 83 bis 85 % erreichbar wäre. Eine Reinheit von 99,3 %, wie sie mit der längsunterteilten Kolonne erhalten wurde, würde eine etwa 17fach höhere Heizleistung erfordern.

Patentansprüche

1. Destillationskolonne zur destillativen Zerlegung eines an einer Zulaufstelle in die Destillationskolonne eintretenden Zulaufproduktes, bestehend aus mehreren Fraktionen, in eine reine Kopf- und eine reine Sumpffraktion und mehrere, vorzugsweise eine oder zwei, im Siedebereich zwischen Kopf- und Sumpffraktion liegende und von Verunreinigungen durch Kopf- und Sumpffraktionen freie oder weitgehend freie Mittelsiederfraktionen, dadurch gekennzeichnet, daß in einem Teilbereich der Destillationskolonne unterhalb und/oder oberhalb der Zulaufstelle in Längsrichtung wirksame Trenneinrichtungen zur Verhinderung einer Quervermischung von Flüssigkeits- und/oder Brüdenströmen angeordnet sind, die die Destillationskolonne in einen Zaulaufteil, in den das Zulaufprodukt eintritt, und einen Entnahmeteil, aus dem die Mittelsiederfraktionen austreten, unterteilen, und daß die in Längsrichtung wirksamen Trenneinrichtungen über so viele Trennstufen ausgeführt sind, daß im Entnahmeteil die von Verunreinigungen durch Kopf- und Sumpffraktionen freien oder weitgehend freien Mittelsiederfraktionen abgezogen werden können.

2. Trenneinrichtungen gemäß Anspruch 1, dadurch gekennzeichnet, daß bei einer Füllkörperkolonne oder einer Kolonne mit Packungen die Trenneinrichtungen als durchgehende Trennbleche zwischen den Kolonnenwänden ausgeführt sind.

3. Trenneinrichtungen gemäß Anspruch 1, dadurch gekennzeichnet, daß bei einer Kolonne mit Regensiebböden die Kolonnenwänden ausgeführt sind, wobei die Höhe der Trennbleche dem Bodenabstand zwischen 2 Regensiebböden entspricht.

4. Trenneinrichtungen gemäß Anspruch 1, dadurch gekennzeichnet, daß bei Bodenkolonnen mit einem oder mehreren Ablaufschächten die Trenneinrichtungen als durchgehende Trennbleche zwischen den Kolonnenwänden ausgeführt sind, wodurch der Raum zwischen den einzelnen Böden und die Ablaufschächte unterteilt werden.

5. Trenneinrichtungen gemäß Anspruch 1, dadurch gekennzeichnet, daß bei Bodenkolonnen mit mehreren Ablaufschächten die Trenneinrichtungen als durchgehende Trennbleche zwischen den Kolonnenwänden ausgeführt sind und jeder dadurch entstehende Teilraum mit mindestens einem Ablaufschacht ausgerüstet ist.

0122367

6. Trenneinrichtungen gemäß Ansprüchen 1 bis 5, dadurch gekennzeichnet, daß die Trenneinrichtungen aus Materialien, die den Wärmedurchgang verringern, gefertigt sind.

7. Trenneinrichtungen gemäß Ansprüchen 1 bis 5, dadurch gekennzeichnet, daß die Trenneinrichtungen derart konstruiert sind, daß sie wärmeisolierend wirken.

8. Trenneinrichtungen gemäß Ansprüchen 1 bis 5, dadurch gekennzeichnet, daß die Trenneinrichtungen mit einer Beheizungs- und/oder Kühlmöglichkeit ausgeführt sind.

Zeichn.

FIG.1

FIG.2

0122367

FIG.3

FIG.4